# EUROPÄISCHE PATENTSCHRIFT

(11) **EP 2 563 300 B1**
(45) Veröffentlichungstag und Bekanntmachung des Hinweises auf die Patenterteilung: **18.09.2013**
(21) Anmeldenummer: 11724521.7
(22) Anmeldetag: 18.03.2011
(51) Int. Cl.: A61F 5/01

(54) **ORTHESE ZUR KORREKTUR EINER BEINFEHLSTELLUNG**
ORTHOSIS FOR CORRECTION OF A VARUS/VALGUS MALALIGNMENT
ORTHÈSE POUR LA CORRECTION D'UNE POSITION DÉFICIENTE DE LA JAMBE

(30) Priorität: 30.04.2010 DE 102010019355
(43) Veröffentlichungstag der Anmeldung: 06.03.2013
(73) Patentinhaber: Otto Bock HealthCare GmbH, 37115 Duderstadt (DE)
(72) Erfinder: DREWITZ, Heiko, 37130 Gleichen (DE)
(74) Vertreter: Lins, Edgar
(86) Internationale Anmeldenummer: PCT/DE2011/000292
(87) Internationale Veröffentlichungsnummer: WO 2011/134446

(56) Entgegenhaltungen:
- EP-A2- 1 508 318
- WO-A1-03/057094
- DE-A1- 4 418 382

## Beschreibung

Die Erfindung betrifft eine Orthese zur Korrektur einer Beinfehlstellung einer Person, mit einer Stützeinrichtung, die in der Frontalebene L-förmig mit einem einen Fuß der Person untergreifenden und einen Kontakt mit einer Lauffläche herstellenden Auflageschenkel und einer seitlich am Bein nach oben ragenden Schienenanordnung ausgebildet ist, die mit einem Befestigungseinrichtung mit dem Unterschenkel der Person verbindbar ist, wobei ein bei Belastung starres Winkelstück am Übergang von dem Auflageschenkel zur Schienenanordnung ausgebildet ist und die Schienenanordnung durch ein etwa in Höhe des Knöchelgelenks angeordnetes Drehgelenk in einen zur seitlichen Anlage am Fuß vorgesehenen Anlageschenkel und in eine zur seitlichen Anlage am Unterschenkel und zur Ausübung eines Drehmoments auf den Unterschenkel vorgesehenen Schiene unterteilt ist.

Die hier angesprochenen Beinfehlstellungen sind eine X-Bein-Stellung oder eine O-Bein-Stellung, die sich durch eine mediale oder laterale Verlagerung der frontalen Mittenachse des Knies ausdrückt.

Es ist bekannt, zur Korrektur einer derartigen Beinfehlstellung eine Orthese zu verwenden, die sich an der Außenseite des Beins mit einem Oberschenkelteil und einem Unterschenkelteil erstreckt, die in Höhe des Kniegelenks durch ein Drehgelenk miteinander verbunden sind. Oberschenkelteil und Unterschenkelteil bilden dabei einen von 180° abweichenden Winkel miteinander, sodass auf das Kniegelenk über die Verbindung des Oberschenkelteils mit dem Oberschenkel und des Unterschenkelteils mit dem Unterschenkel ein Drehmoment zur Korrektur der Beinfehlstellung ausgeübt wird. Durch das Drehgelenk bleibt die Beweglichkeit des Kniegelenks erhalten.

Es ist auch bereits versucht worden, die Korrektur von Beinfehtstellungen durch ein auf das Kniegelenk wirkendes Drehmoment zu korrigieren, das mit Hilfe einer nur am Fuß und Unterschenkel angebrachten Orthese generiert wird.

AT 25437 offenbart hierzu eine Orthese der eingangs erwähnten Art, bei der der Winkel zwischen dem Auflageschenkel und der Schienenanordnung, je nach Korrektur einer O-Bein-Stellung bzw. X-Bein-Stellung, ein spitzer Winkel (< 90°) oder ein stumpfer Winkel (> 90°) ist. Die Wirksamkeit dieser Orthese beruht darauf, dass der Auflageschenkel einen Winkel mit der Lauffläche bildet und somit außermittige Auflagepunkte auf der Lauffläche aufweist. Während des Gehens oder Stehens führt die Belastung mit dem Körpergewicht zur Ausbildung eines Drehmoments, mit dem die Schienenanordnung vom Unterschenkel wegzieht oder gegen den Unterschenkel drückt. Die Schiene und der Anlageschenkel sind dabei bzgl. der Frontalebene winkelstarr miteinander verbunden, da das Drehgelenk in Höhe des Knöchelgelenks lediglich eine Drehbewegung in der Sagittalebene zulässt. Der auf den Unterschenkel wirkende Zug oder Druck ist dabei vom Körpergewicht der die Orthese tragenden Person abhängig. Darüber hinaus hat sich eine derartige Orthese nicht als wirksam herausgestellt, weil die Verschwenkung des Auflageschenkels beim Belasten auf der Lauffläche zu einer Verschenkung des Fußes relativ zum Unterschenkel im Knöchelgelenk führt, wodurch der mit der Orthese angestrebte Effekt weitgehend zerstört wird. Die in der genannten Patentschrift aus dem Jahre 1906 beschriebene Lösung ist aus den genannten Gründen von der Fachwelt als untauglich angesehen worden.

Demgemäß wird die Korrektur der Beinfehlstellungen seit Jahrzehnten mit den eingangs beschriebenen Orthesen vorgenommen, die mit einer Oberschenkelschiene am Oberschenkel und mit einer Unterschenkelschiene am Unterschenkel befestigt sind und ein Drehgelenk in Höhe des Kniegelenks aufweisen, wobei die Stellung der Oberschenkelschiene relativ zur Unterschenkelschiene das für die Korrektur der Beinfehlstellung benötigte Drehmoment aufbringt. Diese Orthesen müssen daher eine erhebliche Größe aufweisen und sind daher aufwendig. Aufgrund Ihrer Größe beeinträchtigen Sie das Erscheinungsbild des Orthesenträgers erheblich.

Der Erfindung liegt daher die Aufgabe zugrunde, eine Orthese zur Korrektur einer Beinfehlstellung einer Person so auszubilden, dass die Orthesen weniger aufwendig und auffällig gestaltet werden kann.

Zur Lösung dieser Aufgabe ist erfindungsgemäß eine Orthese der eingangs erwähnten Art dadurch gekennzeichnet, dass die Schiene als federndes Element ausgebildet ist und dass das Drehmoment aus einer eingestellten Vorspannung des federnden Elements relativ zum Unterschenkel resultiert.

Erfindungsgemäß wird die Korrektur der Beinfehlstellung mit Hilfe einer Orthese vorgenommen, die aus einem Fußteil und einem Unterschenkelteil besteht, die in Höhe des Knöchelgelenks über ein Drehgelenk miteinander verbunden sind. Dabei bildet der Auflageschenkel mit dem Anlageschenkel einen festen Winkel aus. Der Auflageschenkel ist zur im Wesentlichen horizontalen Auflage auf der Lauffläche ausgebildet, sodass sich der Winkel des Auflageschenkels relativ zur Lauffläche bei Belastung mit dem Gewicht der Person nicht oder jedenfalls nicht wesentlich ändert. Das zur Korrektur der Beinfehlstellung aufzubringende Drehmoment resultiert aus der Vorspannung der federnden Schiene.

Der Auflageschenkel kann flächig und parallel zur Lauffläche, beispielsweise in Form einer Sohle, ausgebildet sein. Der Auflageschenkel kann auf seiner zur Lauffläche gerichteten Fläche aber auch leicht ballig ausgeführt sein, um eine geringfügige seitliche Beweglichkeit im Knöchelgelenk, beispielsweise zur Ausbalancierung des Körpergewichts zu erhalten.

Die erfindungsgemäße Orthese blockiert somit nicht durch erzwungenes Abknicken des Fußes gegenüber dem Unterschenkel die Beweglichkeit des Knöchelgelenks. Da das Drehmoment zur Korrektur der Beinfehlstellung aus der federnden Anordnung der Schiene oberhalb des Drehgelenks resultiert, ergibt sich ein kleine Orthese, die im Fuß- und Unterschenkelbereich relativ unauffällig getragen werden kann. Dieser erhält die freie Beweglichkeit des Beines. Die Beweglichkeit des Kniegelenks bleibt ebenfalls vollständig erhalten, da die Orthese nicht über das Kniegelenk hinausragt. Das auf den Unterschenkel ausgeübte Drehmoment führt zu einer leichten Öffnung des Kniespalts in der gewünschten Richtung und ermöglicht somit über Tragdauer der Orthese eine wirksame Korrektur der Beinfehlstellung.

Die Erfindung soll im Folgenden anhand eines in der Zeichnung dargestellten Ausführungsbeispiels näher erläutert werden. Es zeigen:

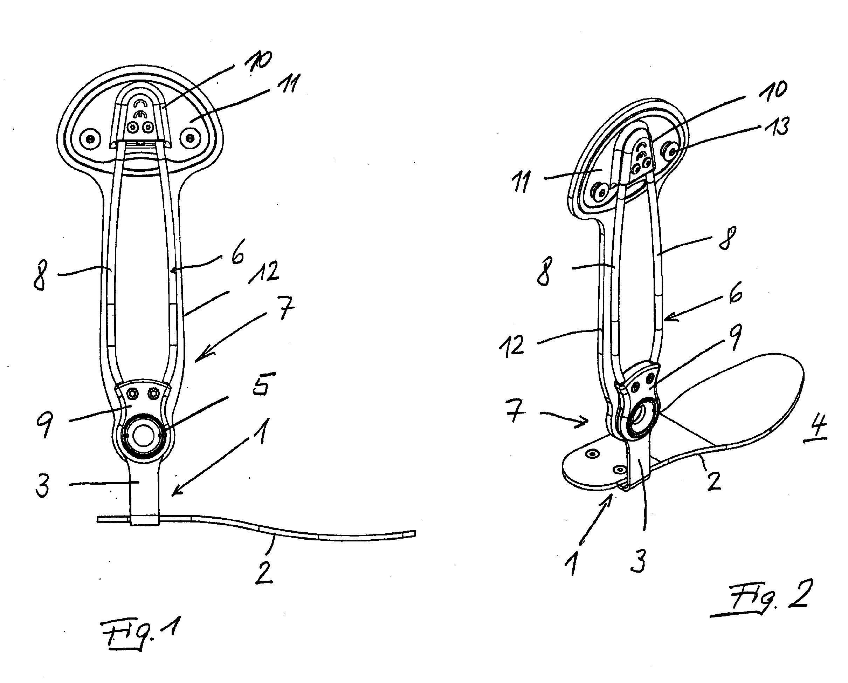

- Figur 1: eine Seitenansicht eines Ausführungsbeispiels einer erfindungsgemäßen Orthese
- Figur 2: eine perspektivische Darstellung der Orthese gemäß Figur 1.

Die in der Zeichnung dargestellte Orthese ist für das rechte Bein eines Orthesenträgers vorgesehen. Sie weist ein Fußteil 1 auf, das von vorne gesehen L-förmig ausgebildet ist und mit einem Auflageschenkel 2 in Form eines Sohlenteils zum Untergreifen des Fußes des Orthesenträgers vorgesehen ist. Auf der lateralen Seite schließt sich an den Auflageschenkel 2 etwa rechtwinklig ein Anlageschenkel 3 an, der zur lateralen Anlage an dem Fuß des Orthesenträgers vorgesehen und ausgebildet ist. Das Fußteil 1 bildet ein massives Teil, dessen Winkel zwischen Auflageschenkel 2 und Anlageschenkel 3 bei Belastung unverändert bleibt. Die Unterseite des Auflageschenkels 2 ist relativ zu einer Lauffläche 4 flächig ausgebildet, sodass sich der Winkel des Fußteils 1 in der Frontalebene unter Gewichtsbelastung beim Gehen oder Stehen relativ zur Lauffläche 4 nicht bzw. nicht wesentlich verändert. Ein elastisches Abrollen der Lauffläche 4 beim Gehen ist in der Sagittalebene dabei möglich und zur Erhöhung des Gehkomforts vorgesehen. Das Fußteil 1 weist am oberen Ende des Anlageschenkels 3 eine Verbindung zu einem Drehgelenk 5 auf über die eine Schiene 6 in der sagittalen Ebene drehbar mit dem Fußteil 1 verbunden ist. Der Anlageschenkel 3, das Drehgelenk 5 und die Schiene 6 bilden somit eine mit dem Auflageschenkel 2 verbundene Schienenanordnung 7, die sich von dem Auflageschenkel 2 nach oben erstreckt und zur seitlichen Anlage an Fuß und Unterschenkel des Orthesenträgers ausgebildet ist.

Die Schiene ist in dem dargestellten Ausführungsbeispiel durch zwei gebogene Rohre 8 gebildet, die sich von einer V-förmigen Aufnahme 9 am Drehgelenk 5 nach oben erstrecken und etwas unterhalb des Knies des Orthesenträgers in eine ebenfalls V-förmige Aufnahme 10 einmünden.

Die Rohre 8 sind in seitlicher Ansicht so gebogen, dass sie in die Aufnahmen 9, 10 mit einem spitzen Winkel zueinander eingesteckt sind und sich ihr Abstand zum mittleren Bereich hin vergrößert. Darüber hinaus sind die Rohre 8 in seitlicher Richtung ebenfalls gebogen ausgebildet, sodass sie beispielsweise von der Aufnahme 9 zunächst zum Unterschenkel hin verläuft, um dann mit einem geringeren Winkel zur Lotrechten nach oben zur Aufnahme 10 geführt zu werden.

Das Drehgelenk 5 befindet sich etwa in Höhe des Knöchelgelenks. Somit ist die in der Zeichnung dargestellte Schiene 6 an die übliche Form eines Unterschenkels angepasst, der im Fesselbereich oberhalb des Knöchelgelenks eine minimale Breite aufweist, um sich dann im Wadenbereich erheblich zu verbreitern. Die dargestellte Orthese entspricht dieser Form, jedoch mit einer Neigung der Schiene 6 nach medial, wodurch eine Vorspannung der federnden Schiene 6 entsteht, mit der die Schiene 6 insbesondere am oberen Ende, also kurz unterhalb des Kniegelenks, auf der lateralen Seite nach medial drückt, um so eine Korrekturkraft für eine Varus-Gonarthrose (O-Bein-Stellung) auszuüben.

Bei der in der Zeichnung dargestellten Ausführungsform wird die durch die Rohre 8 gebildete Schiene 6 durch die Aufnahme 9, 10 stabilisiert, die ein Auseinanderbiegen der Rohrstücke in Richtung der Sagittalebene, also in der dargestellten Breite der Schiene 6, verhindern.

Die obere Aufnahme 10 ist an einer zum Unterschenkel zeigenden Platte 11 befestigt, die für eine Verteilung des Anpressdrucks an den Unterschenkel sorgt.

Die beschriebene tragende Struktur der Orthese wird durch eine Polsterung 12 auf der Innenseite der Schiene 6, des Drehgelenks 5 und der Platte 11 ergänzt, um ein angenehmes Tragen der Orthese an den Körperteilen zu ermöglichen. Nicht dargestellt ist ein Befestigungsriemen, der an Befestigungsknöpfen 13 an der Platte 11 befestigbar und als Klettbandverschluss ausgebildet sein kann, um eine stufenlose Anpassung an den Umfang des Unterschenkels unterhalb des Knies des Orthesenträgers zu ermöglichen.

Das Fußteil 1 wird anderweitig am Orthesenträger fixiert, beispielsweise durch ein gemeinsames Einbringen des Fußteils 1 mit dem Fuß des Orthesenträgers in einen Schuh.

Die dargestellte Orthese dient somit zur Korrektur einer Varus-Gonarthrose (O-Bein-Stellung), indem ein Druck auf den Unterschenkel kurz unterhalb des Kniegelenks mit der Schiene 6 ausgeübt wird. Die Rohre 8 können aber auch so gebogen werden, dass es einen nach lateral geneigten Winkel mit dem Anlageschenkel 3 ausbildet, sodass über den Befestigungsriemen eine Zugkraft auf den Unterschenkel unterhalb des Kniegelenks ausgeübt wird, um so eine Korrekturkraft für eine Valgus-Gonarthrose (X-Bein-Stellung) auszuüben. Hierzu werden die gebogenen Rohre 8 in den Aufnahmen 9, 10 um etwa 180° um ihre Längsachse gedreht.

Grundsätzlich wäre auch möglich, die Orthese medial am Unterschenkel des Orthesenträgers angreifen zu lassen, sodass für eine Valgus-Gonarthrose eine Druckkraft und für eine Varus-Gonarthrose eine Zugkraft ausgeübt wird. Bei einer derartigen Anordnung wird jedoch häufig eine Behinderung des Gehens eintreten, sodass die laterale Anordnung im Allgemeinen zu bevorzugen ist.

Das dargestellte Ausführungsbeispiel lässt erkennen, dass die von der Schiene 6 ausgeübte Korrekturkraft erst oberhalb des Drehgelenks 5 auf den Unterschenkel des Orthesenträgers einwirkt. Demgemäß wird durch die Ausübung der Korrekturkraft die Stellung des Fußes in dem Fußteil 1 bzw. relativ zur Lauffläche 4 nicht beeinflusst, sodass die Korrekturkraft nicht durch eine seitliche Verschwenkung des Fußes relativ zum Unterschenkel kompensiert wird. Vielmehr bleibt der Fuß durch das Fußteil 1 in seinem Winkel zur Lauffläche 4 unverändert, sodass die von der Schiene 6 ausgeübte Korrekturkraft dort wirksam wird, wo sie wirksam werden soll, um den geschädigten medialen (oder lateralen) Bereich im Kniespalt durch die Korrekturkraft zu entlasten.

Die erfindungsgemäße Orthese erlaubt somit erstmalig eine wirksame Aufbringung einer Korrekturkraft bei einer Gonarthrose mit einer Erstreckung nur in den Fußbereich und Unterschenkelbereich des Orthesenträgers, sodass auf ein Oberschenkelteil und ein Kniegelenkteil der Orthese verzichtet werden kann.

## Patentansprüche

1. Orthese zur Korrektur einer Beinfehlstellung einer Person, mit einer Stützeinrichtung, die in der Frontalebene L-förmig mit einem einen Fuß der Person untergreifenden und einen Kontakt mit einer Lauffläche (4) herstellenden Auflageschenkel (2) und einer seitlich am Bein nach oben ragenden Schienenanordnung (7) ausgebildet ist, die mit einem Befestigungseinrichtung (13) mit dem Unterschenkel der Person verbindbar ist, wobei ein bei Belastung starres Winkelstück am Übergang von dem Auflageschenkel (2) zur Schienenanordnung (7) ausgebildet ist und die Schienenanordnung (7) durch ein etwa in Höhe des Knöchelgelenks angeordnetes Drehgelenk (5) in einen zur seitlichen Anlage am Fuß vorgesehenen Anlageschenkel (3) und in eine zur seitlichen Anlage am Unterschenkel und zur Ausübung eines Drehmoments auf den Unterschenkel vorgesehenen Schiene (6) unterteilt ist, **dadurch gekennzeichnet, dass** die Schiene (6) als federndes Element ausgebildet ist und dass das Drehmoment aus einer eingestellten Vorspannung des federnden Elements relativ zum Unterschenkel resultiert.

2. Orthese nach Anspruch 1, **dadurch gekennzeichnet, dass** der Auflageschenkel (2) zur Lauffläche (4) hin mit einer Auflagefläche oder mit mittigen Auflagepunkten ausgebildet ist.

3. Orthese nach Anspruch 1 oder 2, **dadurch gekennzeichnet, dass** die Schiene (6) formgebend gebogene Rohre (8) aufweist, die mit ihren beiden freien Rohrenden in Aufnahmen (9, 10) einmünden.

4. Orthese nach einem der Ansprüche 1 bis 3, **dadurch gekennzeichnet, dass** die Schiene (6) oberhalb des Drehgelenks (5) eine vom Bein des Orthesenträgers ausgesehen konkave Wölbung aufweist, die in eine konvexe Wölbung zur Anpassung an eine Wadenform des Unterschenkels übergeht.

## Claims

1. An orthosis for correcting a leg malalignment of a person, comprising a support apparatus, formed in an L-shaped fashion in the frontal plane with a support limb (2), which engages below a foot of the person and makes contact with a walking surface (4), and with a brace arrangement (7) projecting laterally upward along the leg, which brace arrangement can be connected to the lower leg of the person using an attachment apparatus (13), wherein a bracket which is rigid under stress is formed at the transition from the support limb (2) to the brace arrangement (7) and the brace arrangement (7) is subdivided by a rotary joint (5) which is arranged approximately level with the ankle joint into a contact limb (3), provided for lateral contact on the foot, and a brace (6), provided for lateral contact on the lower leg and for exerting a torque on the lower leg, **characterized in that** the brace (6) is embodied as a resilient element and **in that** the torque results from a preset pretension of the resilient element relative to the lower leg.

2. The orthosis as claimed in claim 1, **characterized in that** the support limb (2) is formed with a support surface or with central support points in the direction of the walking surface (4).

3. The orthosis as claimed in claim 1 or 2, **characterized in that** the brace (6) has tubes (8) which are bent in a shaping manner and, with their two free tube ends, enter holders (9, 10).

4. The orthosis as claimed in one of claims 1 to 3, **characterized in that** the brace (6) has a concave curvature, as seen from the leg of the orthosis wearer, above the rotary joint (5), which curvature merges into a convex curvature for matching to a sural shape of the lower leg.

## Revendications

1. Orthèse pour la correction d'une position déficiente de la jambe d'une personne, comprenant un système de soutien qui est réalisé en forme de L dans le plan frontal avec un bras d'appui (2) qui passe au-dessous du pied de la personne et qui établit un contact avec une surface de marche (4), et avec un agencement d'éclisse (7) qui se dresse vers le haut latéralement au niveau de la jambe et qui est susceptible d'être relié à la jambe de la personne avec un système de fixation (13), dans laquelle une pièce en équerre rigide sous charge est réalisée à la transition du bras d'appui (2) vers l'agencement d'éclisse (7) et l'agencement d'éclisse (7) est subdivisé par une articulation rotative (5) agencée approximativement à la hauteur de l'articulation de la cheville en un bras d'appui (3) prévu pour venir en appui latéralement contre le pied et en une éclisse prévue pour venir en appui latéral contre la jambe et pour exercer un couple de rotation sur la jambe, **caractérisée en ce que** l'éclisse (6) est réalisée comme un élément avec effet ressort, et **en ce que** le couple de rotation résulte d'une précontrainte établie de l'élément avec effet ressort par rapport à la jambe.

2. Orthèse selon la revendication 1, **caractérisée en ce que** le bras d'appui (2) est réalisé, en direction de la surface de marche (4), avec une surface d'appui ou avec des points d'appui au milieu.

3. Orthèse selon la revendication 1 ou 2, **caractérisée en ce que** l'éclisse (6) comprend des tubes (80) cintrés de manière à les mettre en forme, qui débouchent avec leurs deux extrémités libres dans des logements (9, 10).

4. Orthèse selon l'une des revendications 1 à 3, **caractérisée en ce que** l'éclisse (6) comporte, au-dessus de l'articulation rotative (5), un bombement concave, vu depuis la jambe du porteur de l'orthèse, qui se transforme en un bombement convexe pour s'adapter à la forme du mollet de la jambe.
